# EUROPEAN PATENT SPECIFICATION

(11) **EP 4 031 002 B1**
(45) Date of publication and mention of the grant of the patent: **28.10.2026**
(21) Application number: 20865400.4
(22) Date of filing: 16.09.2020
(51) Int. Cl.: A61B 5/103, A61B 5/107, A61B 5/11, G01B 7/30, A61B 5/00

(54) **WEARABLE DEVICE FOR COUPLING TO A USER, AND MEASURING AND MONITORING USER ACTIVITY**
AM KÖRPER TRAGBARE VORRICHTUNG ZUM ANKOPPELN AN EINEN BENUTZER SOWIE MESSUNG UND ÜBERWACHUNG DER BENUTZERAKTIVITÄT
DISPOSITIF PORTABLE POUR LE COUPLAGE À UN UTILISATEUR, ET MESURE ET SURVEILLANCE DE L'ACTIVITÉ DE L'UTILISATEUR

(30) Priority: 17.09.2019 US 201962901464 P; 17.09.2019 US 201962901411 P; 23.09.2019 US 201962904013 P; 07.10.2019 US 201962911515 P; 18.03.2020 US 202062991295 P; 10.09.2020 US 202017017062
(43) Date of publication of application: 27.07.2022
(73) Proprietor: ROM Technologies, Inc., Brookfield, CT 06804 (US)
(72) Inventor: HACKING, S. Adam, Brookfield, CT 06804 (US); TAMRAGOURI, Sucheta, Brookfield, CT 06804 (US); COTE, Jeff, Brookfield, CT 06804 (US); LIPSZYC, Daniel, Brookfield, CT 06804 (US); TAVERAS, Mikael, Brookfield, CT 06804 (US)
(74) Representative: Gevers Patents
(86) International application number: PCT/US2020/051091
(87) International publication number: WO 2021/055491

(56) References cited:
- WO-A1-2005/018453
- WO-A1-2018/081795
- US-A1- 2003 083 596
- US-A1- 2003 083 596
- US-A1- 2007 276 296
- US-A1- 2008 202 233
- US-A1- 2016 140 319
- US-B1- 8 607 465
- US-B1- 8 607 465
- US-B2- 7 406 003
- US-B2- 7 726 034

## Description

### CROSS-REFERENCE TO RELATED APPLICATIONS

This application claims priority to and the benefit of:
U.S. Pat. App. No. 17/017,062 (Atty. Dkt. 91346-905), filed September 10, 2020,
U.S. Prov. Pat. App. No. 62/991,295 (Atty. Dkt. 91346-1010), filed March 18, 2020,
U.S. Prov. Pat. App. No. 62/911,515 (Atty. Dkt. 91346-1700), filed October 7, 2019,
U.S. Prov. Pat. App. No. 62/904,013 (Atty. Dkt. 91346-1500), filed September 23, 2019,
U.S. Prov. Pat. App. No. 62/901,464 (Atty. Dkt. 91346-900), filed September 17, 2019, and
U.S. Prov. Pat. App. No. 62/901,411 (Atty. Dkt. 91346-1000), filed September 17, 2019.

### TECHNICAL FIELD

This disclosure generally relates to wearable devices and, in particular, to coupling a wearable device to a user and measuring and monitoring activity of the user.

### BACKGROUND

A patient often requires physical therapy to recover from surgery, such as a knee replacement surgery. The physical therapy can include exercise to increase the patient's strength, flexibility and stability. If a patient over-extends his or her muscles or joints, the muscles or joints, surrounding tissues or repaired tissues may become further injured. If a patient does not exercise his or her muscles or joints to gain the appropriate range of motion, the joint may become stiff and require additional surgery. Measuring and monitoring the range of motion during physical therapy can help prevent further injury to the patient and result in a faster recovery time.

A goniometer is an instrument that can be used to measure ranges of motion or joint angles of a patient's body. A standard goniometer consists of a stationary arm that cannot move independently, a moving arm attached to a fulcrum in the center of a body, and the body being a protractor of which 0 to 180 or 360 degrees are drawn. The stationary arm is attached to one limb or part of the patient's body (e.g., a thigh) and the moving arm is attached to another limb or part of the patient's body (e.g., a lower leg). The fulcrum can be a rivet or screw-like device at the center of the body that allows the moving arm to move freely on the body of the device in order for a clinician to obtain a measurement of the angle of movement of the patient's joint (e.g., a knee). The measurements can be used to track progress in a rehabilitation program. Each time a patient has a rehabilitation session, the clinician places and hold, or attaches the goniometer device onto the patient, for example, using straps. The patient may have different clinicians setting up the goniometer device and measuring the joint movement. Based on the experience of the clinician, or other person, the goniometer device may be attached onto different locations on the patient, which can affect the accuracy and reproducibility of the measurements. The accuracy of the repeated measurements may also be compromised due to issues with or the sensitivity of the device.

Related technologies are known from WO 2005/018453 A1 and US 2003/083596 A1.

### SUMMARY

The present invention relates to a system for measuring an angle of a joint of a user showing the technical characteristics of the first independent claim. The system includes a center hub, a first arm, a second arm, a magnet, a sensor, a printed circuit board (PCB) and an outward notch. The center hub includes a first hub and a second hub. The first hub and the second hub are configured to rotate 360 degrees relative to each other. The first arm is configured for attachment to a first limb portion of the user at a first outer end and to the first hub at a first inner end. The second arm is configured for attachment to a second limb portion of the user at a second outer end and to the second hub at a second inner end, wherein the first hub is pivotally coupled to the second hub. The magnet is coupled to the second hub. The sensor is disposed in the center hub and configured to detect a rotation of the magnet. The PCB is removably disposed in the center hub. The PCB has an inward notch. The outward notch is coupled to the first hub. The outward notch is configured to couple with the inward notch to align the sensor and the magnet.

### BRIEF DESCRIPTION OF THE DRAWINGS

The disclosure is best understood from the following detailed description when read in conjunction with the accompanying drawings. It is emphasized that, according to common practice, the various features of the drawings are not to scale. On the contrary, the dimensions of the various features are arbitrarily expanded or reduced for clarity.

For a detailed description of example embodiments, reference will now be made to the accompanying drawings in which:
FIGS. 1A and 1B are perspective views of a wearable device for measuring and recording movement in accordance with aspects of the present disclosure.
FIGS. 2A and 2B are top and bottom perspective views of a goniometer in accordance with aspects of the present disclosure.
FIGS. 3A and 3B are top and side views of a goniometer in accordance with aspects of the present disclosure.
FIG. 4 is an exploded view of a goniometer in accordance with aspects of the present disclosure.
FIGS. 5A-C are side views of the goniometer with its arms rotating in accordance with aspects of the present disclosure.
FIG. 6 is a perspective view of the goniometer with its arms twisting in accordance with aspects of the present disclosure.
FIGS. 7A-D are top schematic views of the attachment in accordance with aspects of the present disclosure.
FIGS. 8A-D are views of an attachment in accordance with aspects of the present disclosure.
FIGS. 9A-D are views of first and second layers of the attachment in accordance with aspects of the present disclosure.
FIGS. 10A-D are views of a pod of the attachment in accordance with aspects of the present disclosure.
FIGS. 11A and 11B are perspective and cross-sectional views of a coupling apparatus in accordance with aspects of the present disclosure.
FIGS. 12A and 12B are perspective views of the attachment and a goniometer attachment in accordance with aspects of the present disclosure.
FIG. 13 is a cross sectional view of the goniometer in accordance with aspects of the present disclosure.
FIGS. 14A-C are perspective and top views of a center hub of the goniometer closed, open, and with components removed in accordance with aspects of the present disclosure.
FIGS. 15A and 15B are top and bottom views of a printed circuit board (PCB) in accordance with aspects of the present disclosure.
FIGS. 16A and 16B are graphs illustrating test data in accordance with aspects of the present disclosure.
FIG. 17 is a perspective view of the alignment device, with the alignment device shown aligned relative to the center of the joint of the user.
FIG. 18 is a perspective view of the user illustrating the markings on the skin of the user at the center of the joint, and on the opposing limb portions.
FIG. 19 is a perspective view illustrating the attachments being aligned with the markings on the opposing limb portions.
FIG. 20 is a perspective view illustrating the wearable device being coupled to the attachment, and aligned relative to the center of the joint.
FIG. 21 is a perspective view illustrating an alternative embodiment of the alignment device, and illustrating the light beams at the center of the joint and on the opposing limb portions, which are configured to assist the clinician in aligning the attachments and wearable device relative to the center of the joint.
FIG. 22 is a perspective view illustrating yet another alternative embodiment of the alignment device, and illustrating the diodes at the center of the joint, on the opposing limb portions, and on the wearable device.
FIGS. 23A-23E are various views of an embodiment of a pedometer that may be coupled to the goniometer.

### NOTATION AND NOMENCLATURE

Various terms are used to refer to particular system components. Different companies may refer to a component by different names - this document does not intend to distinguish between components that differ in name but not function. In the following discussion and in the claims, the terms "including" and "comprising" are used in an open-ended fashion, and thus should be interpreted to mean "including, but not limited to...." Also, the term "couple" or "couples" is intended to mean either an indirect or direct connection. Thus, if a first device couples to a second device, that connection may be through a direct connection or through an indirect connection via other devices and connections.

The terminology used herein is for the purpose of describing particular example embodiments only, and is not intended to be limiting. As used herein, the singular forms "a," "an," and "the" may be intended to include the plural forms as well, unless the context clearly indicates otherwise. The method steps, processes, and operations described herein are not to be construed as necessarily requiring their performance in the particular order discussed or illustrated, unless specifically identified as an order of performance. It is also to be understood that additional or alternative steps may be employed.

The terms first, second, third, etc. may be used herein to describe various elements, components, regions, layers and/or sections; however, these elements, components, regions, layers and/or sections should not be limited by these terms. These terms may be only used to distinguish one element, component, region, layer or section from another region, layer or section. Terms such as "first," "second," and other numerical terms, when used herein, do not imply a sequence or order unless clearly indicated by the context. Thus, a first element, component, region, layer or section discussed below could be termed a second element, component, region, layer or section without departing from the teachings of the example embodiments. The phrase "at least one of," when used with a list of items, means that different combinations of one or more of the listed items may be used, and only one item in the list may be needed. For example, "at least one of: A, B, and C" includes any of the following combinations: A, B, C, A and B, A and C, B and C, and A and B and C. In another example, the phrase "one or more" when used with a list of items means there may be one item or any suitable number of items exceeding one.

Spatially relative terms, such as "inner," "outer," "beneath," "below," "lower," "above," "upper," "top," "bottom," and the like, may be used herein. These spatially relative terms can be used for ease of description to describe one element's or feature's relationship to another element(s) or feature(s) as illustrated in the figures. The spatially relative terms may also be intended to encompass different orientations of the device in use, or operation, in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptions used herein interpreted accordingly.

### DETAILED DESCRIPTION

The following discussion is directed to various embodiments of the invention. Although one or more of these embodiments may be preferred, the embodiments disclosed should not be interpreted, or otherwise used, as limiting the scope of the disclosure, including the claims. In addition, one skilled in the art will understand that the following description has broad application, and the discussion of any embodiment is meant only to be exemplary of that embodiment, and not intended to intimate that the scope of the disclosure, including the claims, is limited to that embodiment. The invention is defined by the appended claims.

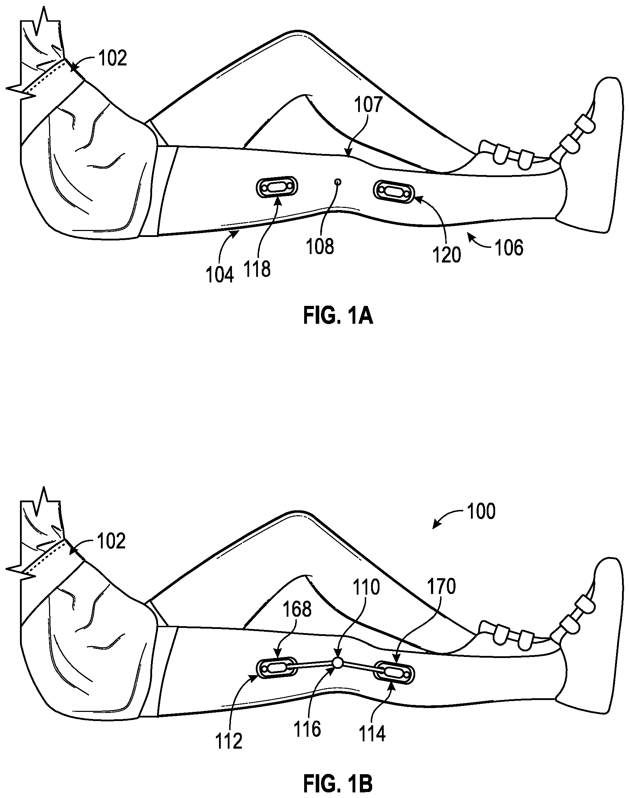

In accordance with aspects of the present disclosure, FIGS. 1A and 1B illustrate an exemplary system or wearable device 100 for measuring and recording flexion and extension at a joint 107 of a user 102. The wearable device 100 comprises first and second coupling apparatuses, or attachments, 112, 114, 118, 120 (hereinafter referred to as first and second attachments 118, 120) and may be configured to be removably coupled to the user 102 at opposing limb portions 104, 106 of the joint 107. For example, and as illustrated in FIGS. 1A and 1B, the first and second attachments 118, 120 may be coupled to a leg of a user at opposing limb portions 104, 106, e.g., thigh 104, and calf 106, of the knee or joint 107 of the user 102. As will be appreciated by those of skill in the art, the first and second attachments 118, 120 may be coupled to the user at opposing limb portions of any other joint of a patient or user 102. It is further contemplated that the wearable device 100 may be utilized for measuring flexion and extension of joints in animals, a joint of a robot, or any other desired joint or joint equivalent.

To position the wearable device 100 relative to the joint 107, a person, such as a clinician, may identify a joint center 108, where the joint center 108 may be used to align the wearable device 100 to the joint 107. The clinician may use an alignment device 300 to identify and mark the joint center 108. For example, the alignment device 300 may be used to mark the skin of the user 102 at the joint center 108 with a marker, pen, or any other desired tool. Further, the alignment device 300 may be used to identify and mark positions at opposing limb portions 104, 106 for the first and second attachments 118, 120 relative to the joint center.

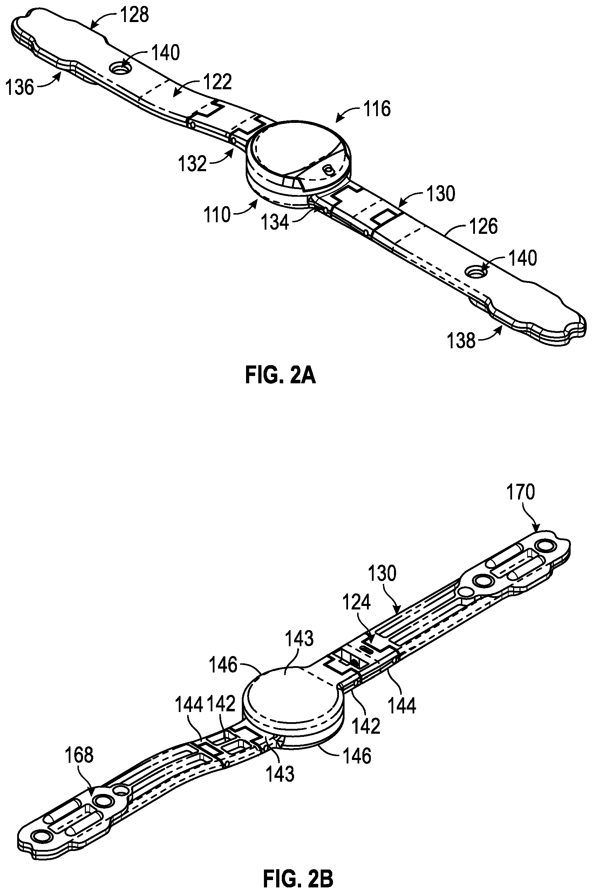

With reference to FIGS. 2A-6, the wearable device 100 comprises an exemplary device or apparatus 110, such as a goniometer. Hereinafter, the device or apparatus 110 may be referred to as a goniometer 110. The goniometer 110 is configured to measure the angular flexion and extension at the joint 107 of the user 102. The goniometer 110 has a top 122, a bottom 124, and opposing sides 126. The goniometer 110 may comprise a center hub 116 aligned coaxially with an axis A, and first and second arms 128, 130, wherein the arms 128, 130 couple to, and are rotatable about, the axis A and the center hub 116. More specifically, first and second inner ends 132, 134 of the respective arms 128, 130 couple to the center hub 116. The arms 128, 130 extend outwardly from the center hub 116 to respective first and second outer ends 168, 170. In an alternative embodiment, the arms 128, 130 may be integrally formed with the center hub 116. Embodiments of the goniometer can enable various rotational or pivotal motions, such as with an axle, a rack and pinion system, etc.

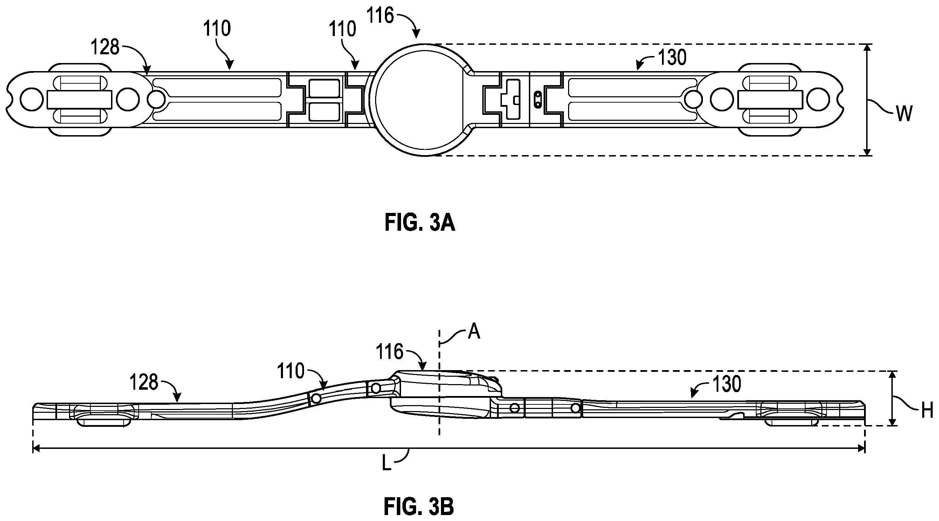

With reference to FIGS. 3A and 3B, the goniometer 110 can have a length L that extends from the first outer end 136 to the second outer end 138. The length L can vary depending on the relative position of the first and second arms 128, 130. For example, a maximum length L of the goniometer 110 may be measured when the arms 128, 130 are positioned opposing one another. Whereas, when the arms 128, 130 are positioned parallel, and respectively directly above and below one another, a minimum length L of the goniometer 110 may be measured between the outer ends 136, 138 to an opposite side of the center hub 116. A width W of the goniometer may be measured as a diameter of the center hub 116. Further, a height H of the goniometer 110 may be measured from a bottom of the second arm 130 to a top of the first arm 128, or to a top of the center hub 116, whichever is greater.

In an exemplary embodiment, the center hub 116 comprises a first or upper hub 146 and a second or lower hub 148. The hubs 146, 148 are coaxially aligned with one another, and with axis A. Moreover, the hubs 146, 148 are configured to rotate about the axis A for 360 degrees, and relative to one another. Further, each of the hubs 146, 148 may have a link arm 143 for coupling between the hubs 146, 148 and the respective arms 128, 130. For example, the first arm 128 may be coupled to the link arm 143 of the first hub 146, and the second arm 130 may be coupled to the link arm 143 of the second hub 148.

In operation, embodiments of the arms 128, 130 may rotate, pivot, flex or extend relative to the center hub 116. This design can account for the complex motion of a joint, slippage of the joint, and the broad range of shapes and sizes of the patient's joint 107. In addition, this design can maintain the position of the center hub relative to the joint center 108. Embodiments of the device can enable freedom of motion in many planes but not in the rotational plane of the joint. This enables the device to fit many different people but still make accurate measurements.

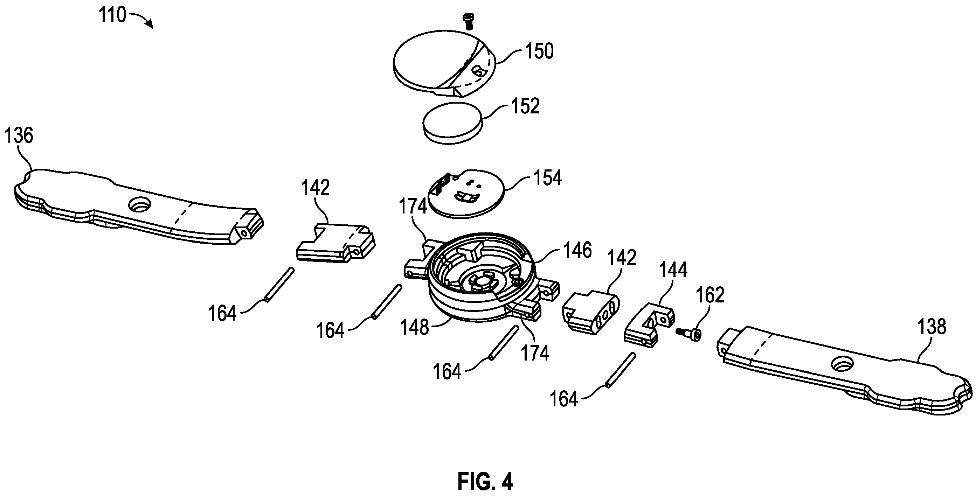

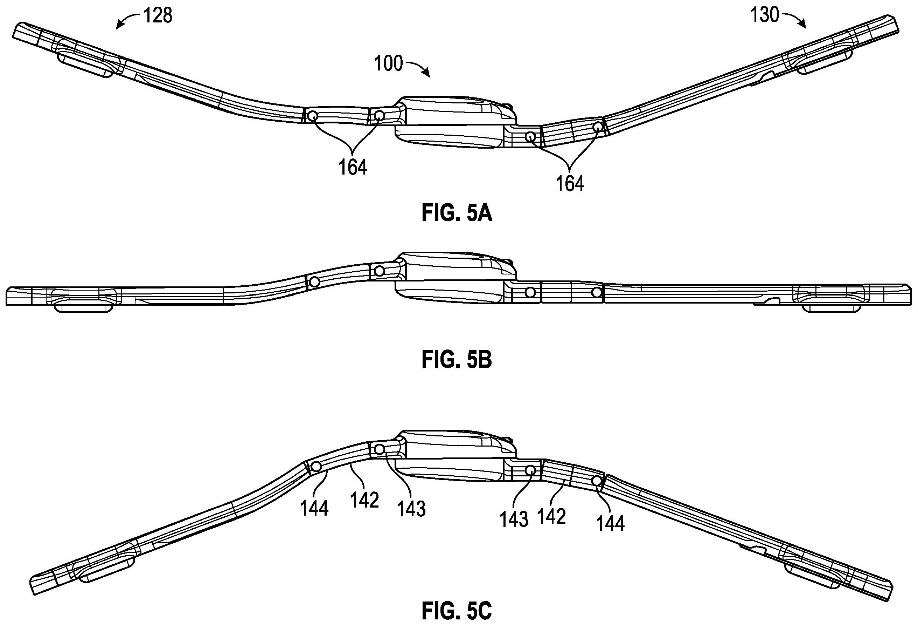

More specifically, and as best illustrated in FIG. 4, the first and second arms 128, 130 may each include an inner link 142 disposed adjacent to the respective inner ends 132, 134, and an outer link 144 disposed between the inner link 142 and the outer ends 168, 170. With reference to FIGS. 5A-C, the inner link 142 may couple to the link arm 143 in order to couple between respective arms 128, 130 and hubs 146, 148. The inner link 142 may be configured to facilitate the pivot, flex, or extension of the respective arm 128, 130 relative to the center hub 116. A pin 164 may be used to couple the inner link 142 and the link arm 143 of each arm 128, 130 to allow for the pivot, flexion, or extension of the respective arms 128, 130. The pin 164 may be disposed perpendicular to the length of the arms 128, 130.

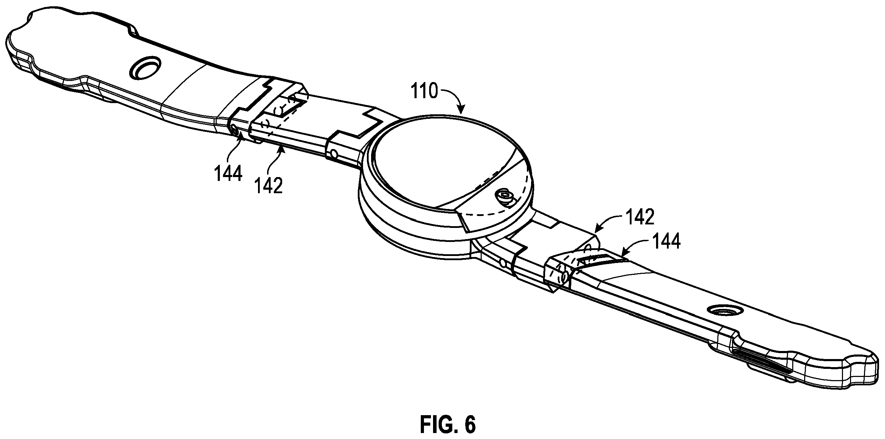

The outer link 144 may couple to the inner link 142 and respective outer ends 136, 138. With reference to FIG. 6, the outer link 144 may be configured to couple to the inner link 142 to facilitate rotation of the respective arms 128, 130 relative to the center hub 116. A screw 162 may be configured to couple the outer link 144 to the inner link 142 to facilitate rotation of the respective arms 128, 130 about the screw 162, all relative to the center hub 116. The screw 162 may align parallel with the length of the respective arm 128, 130. It is to be appreciated that the first and second arms 128, 130 may rotate +/- eighteen degrees, or any other desired amount, in one or more directions. Further yet, and with reference to FIGS. 5A-C, the outer link 144 may be configured to couple to the respective outer ends 136, 138 to facilitate further pivot, flexion, or extension of the respective arms 128, 130 relative to the center hub 116. A pin 164 may be used to couple the outer link 144 and the respective outer ends 136, 138 to allow for further pivot, flexion, or extension, of the respective arms 128, 130 relative to the center hub 116. The pin 164 may be disposed perpendicular to the length of the respective arms 128, 130.

The first and second outer ends 136, 138 may comprise first and second goniometer attachments 168, 170, which may be integral with, or coupled to the respective outer ends 136, 138. It is to be appreciated the goniometer attachments 168, 170 may couple, or be integral with, the arms 128, 130 at any desired location, or in any desired configuration. The first and second goniometer attachments 168, 170 may be configured to removably couple with the attachments 118, 120. Further, each goniometer attachment 168, 170 can comprise one or more bosses 200, and one or more magnets 158 positioned next to the bosses 200 to facilitate the coupling and alignment of the goniometer attachments 168, 170 and the attachments 118, 120. The bosses 200 and magnets 158 further facilitate the alignment of the goniometer 110 relative to the attachments 118, 120. The arms 128, 130 may also include one or more arm alignment holes 140 configured to align with the attachments 118, 120, or an alignment mark on a user 102. The arms 128, 130 may further have one or more wings 202 that extend from a side 126 of the goniometer 110, such as from the first or second goniometer attachments 168, 170. The wings 202 can be formed from or coupled to the first or second goniometer attachments 168, 170. The wings 202 can be a tab or have any other desired shape. The wings 202 may be configured to assist a user in moving the arms 128, 130 of the goniometer perpendicularly relative to the attachments to facilitate uncoupling the goniometer 110 from the attachments 118, 120 without uncoupling the attachments 118, 120 from the user 102.

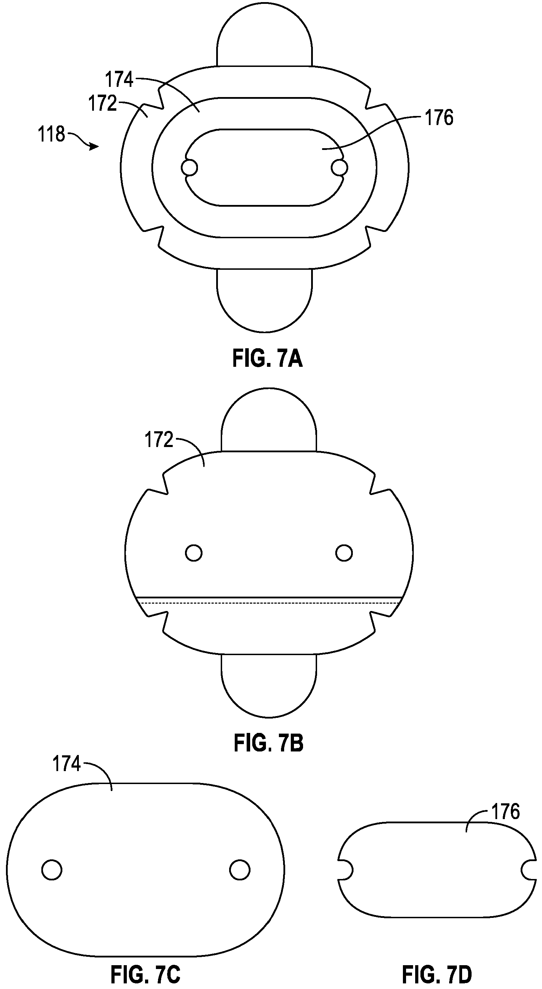

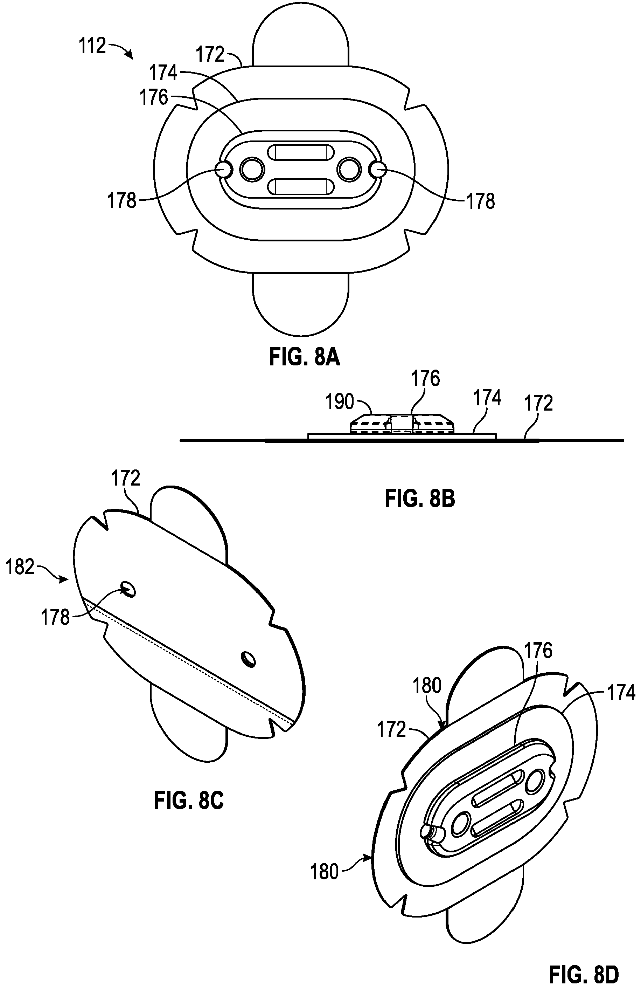

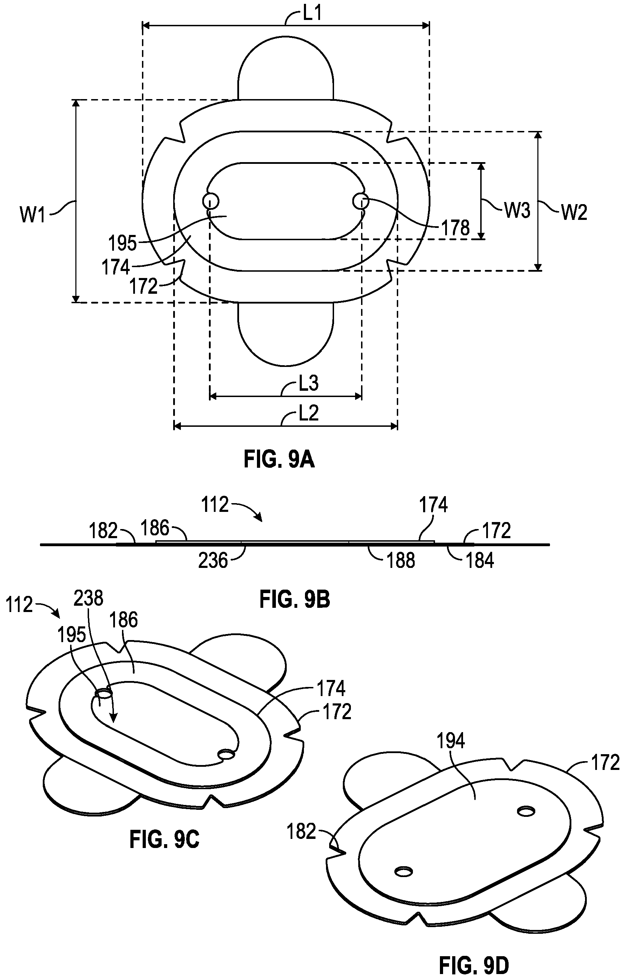

With reference to FIGS. 7-9, the attachments 118, 120 may comprise first and second layers 172, 174 and a pod 176 coupled together with one another. When coupled to one another, each of the layers 172, 174 and the pod 176 may be concentric with one another. The first attachment 118 may be the same as and interchangeable with the second attachment 120. The first layer 172, the second layer 174, and the pod 176 may be generally oval-shaped or any other desired shape. The first layer 172 may be larger than the second layer 174, and the second layer 174 may be larger than the pod 176. Further, the first layer 172 may be thinner than the second layer 174, and the pod 176 can be thicker than the first and second layers 172, 174.

The first layer 172 may have a top 182 and a bottom 184, and may be formed from a pad, coated paper, plastic, woven fabric, latex, or any other desired material. For example, the top 182 may be formed from a pad and the bottom 184 may comprise an adhesive material 236, such as a medical-grade adhesive or other suitable material. The adhesive material 236 couples to the skin of the user 102 to couple the attachments 118, 120 to the user 102. Further, the top 182 may also have an adhesive layer 194, which may be smaller in area than the first layer 172. Further, the adhesive layer 194 can be less than or equal to the area of the second layer 174. The adhesive layer 194 may be ovular in shape, and define one or more notches or voids in an outer periphery. Further, the first layer 172 can define notches 180 in an outer periphery for assisting in aligning the first attachment 118 relative to a predetermined location, or mark, on the user 102. The notches 180 can be v-shaped or have any other desired shape. Further yet, the first layer 172 may define a pair of voids or alignment holes 178, which may assist in the alignment of the first attachment 118 relative to a predetermined location, or mark, on the user 102. The alignment holes 178 and notches 118 of the first layer 172 may be the same as, or aligned with, the notches or voids of the adhesive layer 194.

The second layer 174 may have a top 186 and a bottom 188, and may be formed of a foam material or any other desired material. The second layer 174 may couple to the adhesion layer 194 of the first layer 172. To prevent uncoupling, the foam material of the second layer 174 may dampen forces between the goniometer 110 and the attachments 118, 120. The top 186 of the second layer 174 may also have an adhesive layer 195 on an upper surface 238 of the top 186, which may be smaller in area than the area of the pod 176. The adhesive layer 195 may have an ovular shape, or any other desired shape. Further, the adhesive layer 195 and the second layer 174 may have one or more holes, or one or more cutouts that may be aligned with the alignment hole 178 of the first layer 172 to align the adhesive layer 195 and the second layer 174 with the first layer 172. The adhesive layers 194, 195 can be formed of an adhesive material or any other desired coupling material, such as a hook- or a loop-type material. The first layer 172 can have a length L1 and a width W1. The second layer 174 can have a length L2 and a width W2. The adhesive layer 195 can have a length L3 and a width W3.

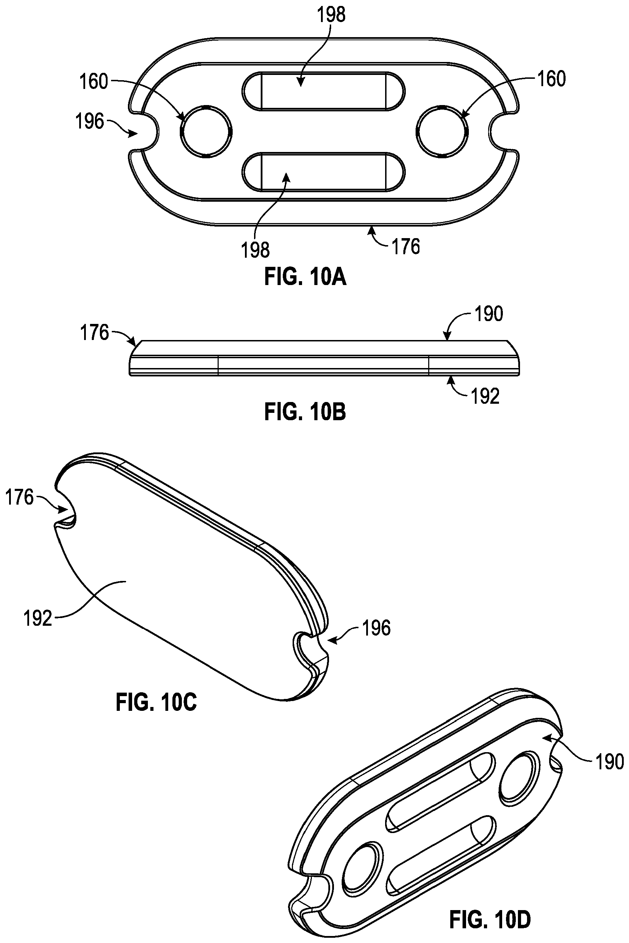

As illustrated in FIGS. 10A-D, the pod 176 has a top 190 and a bottom 192. The pod 176 can include one or more notches 196. For example, notches 196 can be located at opposing ends of the pod 176. The notches 196 can be used for alignment of the first coupling apparatus 112, the pod 176, or any other desired feature of the wearable device 100. The pod 176 can include one or more magnets 160. The magnet 160 may be a neodymium magnet or any other desired magnet. The pod 176 can have a recess for housing the magnet 160. The magnet 160 can be circular or any other desired shape. Two magnets 160 can be disposed in the pod 176 at opposing ends of the pod 176. The pod 176 can be sized to be received by and detachably coupled to the upper surface 238 of the second layer 174.

More specifically, the pod 176 has an underside, such as the bottom 192, which may couple to the upper surface 238 of the second layer 174. The bottom 192 can have one or more hooks or loops, to couple to the upper surface 238. Alternatively, the upper surface 238 and the bottom 192 may comprise an adhesive material to facilitate the detachable coupling between the upper surface 238 and the pod 176. The top 190 of the pod 176 may have one or more recesses 198. The recess 198 may be ovular in shape or have any other desired shape. The recesses 198 may also have tapered edges to assist a user 102 in uncoupling from the pod 176 by moving the arms 128, 130 perpendicularly relative to the pod 176. Two recesses 198 may be formed in the pod 176 at opposing ends or sides of the pod 176.

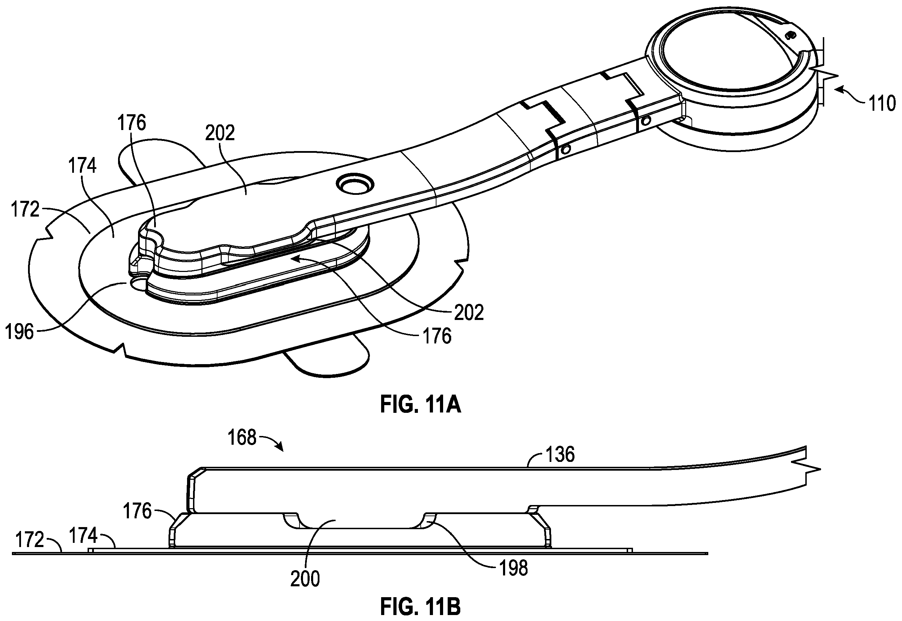

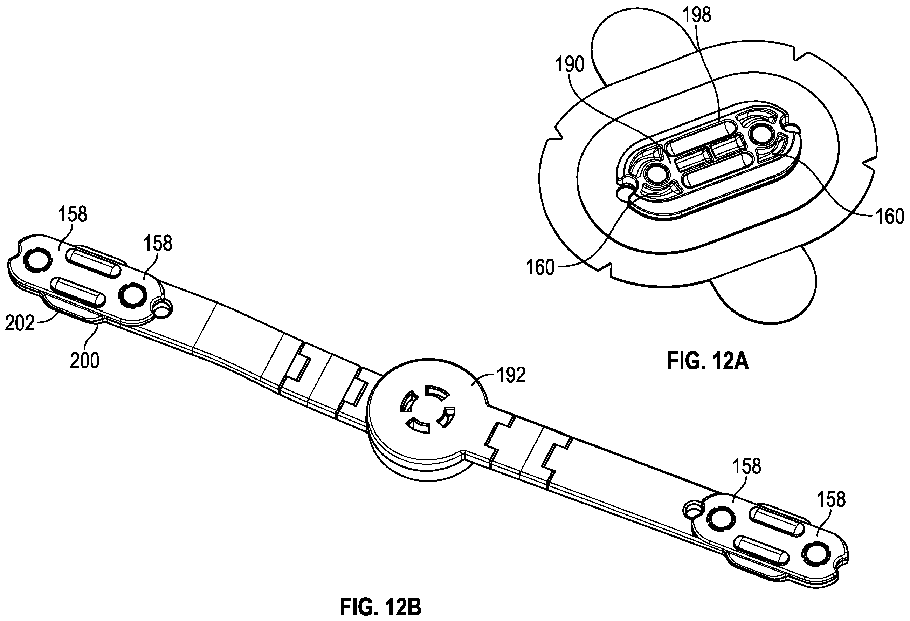

With reference to FIGS. 11-12, the recesses 198 are sized to receive the bosses 200 to align the goniometer 110 relative to the attachments 118, 120. Moreover, the recesses 198 are sized to allow slight movement of the bosses 200 to compensate for slight translational movement of the joints 128, 130 while the goniometer 110 is worn by the user 102. This movement may reduce the stress between, and prevent uncoupling of, the attachments 118, 120 to the skin, clothing, brace, or any other desired location on the user 102.

When the user 102 moves the first or second limb portions 104, 106, the first or second arms 128, 130 move or rotate with the first and second hubs 146, 148. The goniometer 110 can measure the rotation of joint 107 by measuring the angle between the first and second hubs 146, 148. To achieve this, and with reference to FIGS. 4, 13-15, the center hub 116 defines an opening for receiving and containing a printed circuit board (PCB) 152, a sensor 216, a retaining ring 154, a magnet 156, or any other components of the goniometer 110 which cooperate with one another to measure relative angular movement between the arms 128, 130. More specifically, the first and second hubs 146, 148 may define the opening of the center hub 116.

For enclosing the opening, a cover 150 may be attached to the center hub 116, and more specifically to the first hub 146. The cover 150 can be detachably coupled to the first hub 146 or any other desired location. The cover 150 can also be configured to inhibit movement of the PCB 152 and other components located within the center hub 116. For example, when the cover is closed, a bottom portion of the cover 150 may apply direct or indirect pressure to the PCB 152. The cover 150 may have a snap mechanism 226, such as a finger snap or any other desired mechanism, configured to attach and detach the cover to the center hub 116.

The magnet 156 may couple to the second hub 148, and the sensor 216 also disposed in the center hub 116 is configured to detect rotation of the magnet 156. The sensor 216 can be configured to measure the rotation of the magnet 156 to a sensitivity up to one-hundredth of a degree, or to any other desired sensitivity.

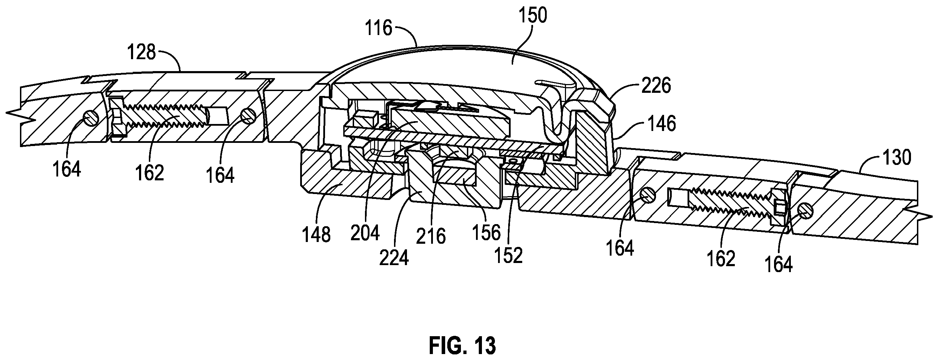

As illustrated in FIGS. 13-14C, the center hub 116 includes the first hub 146 positioned rotatably above the second hub 148. An outward notch 208 may be coupled to the first hub 146, or any other desired location. The outward notch 208 may be formed with the first hub 146 or attached to the first hub 146. The PCB 152 may be removably disposed in the center hub 116. The PCB 152 may have an inward notch 210. The outward notch 208 may be configured to couple with the inward notch 210 to align the sensor 216 and the magnet 156. The alignment can restrict movement of the PCB 152 within the center hub 116.

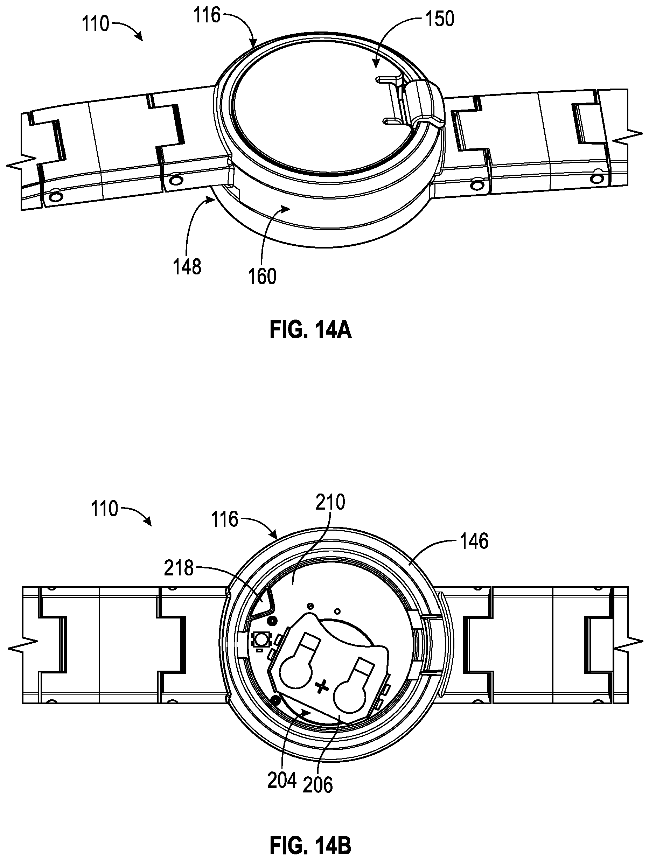

When the cover 150 is removed, the PCB 152 may be accessed. FIG. 14B illustrates a battery housing 206 coupled to the PCB 152. The battery housing 206 may be attached to a top side 212 of the PCB 152. The battery housing 206 may be formed of conductive metal or any other desired material. When the cover 150 is attached to the first hub 146, the cover 150 may apply pressure to the battery housing 206, which may secure PCB 152 within the center hub 116. A battery 204 may be detachably coupled to the battery housing 206. The battery 204 may be a lithium ion battery or any other desired battery or power source. The battery housing 206 may have tabs or any other desired contact points for conduction with the battery 204. The battery 204 may be removed from the battery housing 206 to turn the goniometer 110 off or for replacement of the battery 204. For example, when the cover 150 is opened or removed from the first hub 146, the PCB 152 may be removed from the center hub 116 and the battery 204 may be replaced. To maintain calibration of the goniometer 110 after a battery replacement, the inward notch 210 of the PCB 152 is configured for receiving the outward notch 208 of the first hub 146.

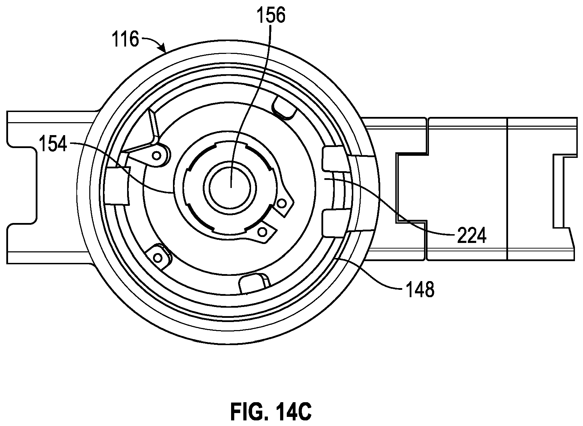

In FIG. 14C, the cover 150 and the PCB 152 are removed from the center hub 116. As shown, the second hub 148 may include a magnet housing 224. The magnet housing 224 may be located in the center of the second hub 148 or any other desired location. The magnet housing 224 may be coupled to the PCB 152 using a weld, an adhesive, a tack, or any other desired attachment. The magnet housing 224 may be configured to receive the magnet 156. The magnet 156 may be configured to have north and south polarity within the center hub 116 or any other desired polarity. The magnet housing 224 may include a lip at a top of the magnet housing 224 or have any other desired configuration. The retaining ring 154 may be configured to couple the magnet 156 to the second hub 148. The retaining ring 154 may be coupled to the second hub 148 around the magnet housing 224. For example, the retaining ring 154 may be positioned between the lip or top of the magnet housing 224 and the second hub 148. The retaining ring 154 may secure the magnet within the magnet housing 224. The retaining ring 154 may move with the second hub 148 and the magnet 156 as the second hub 148 rotates with the second arm 130.

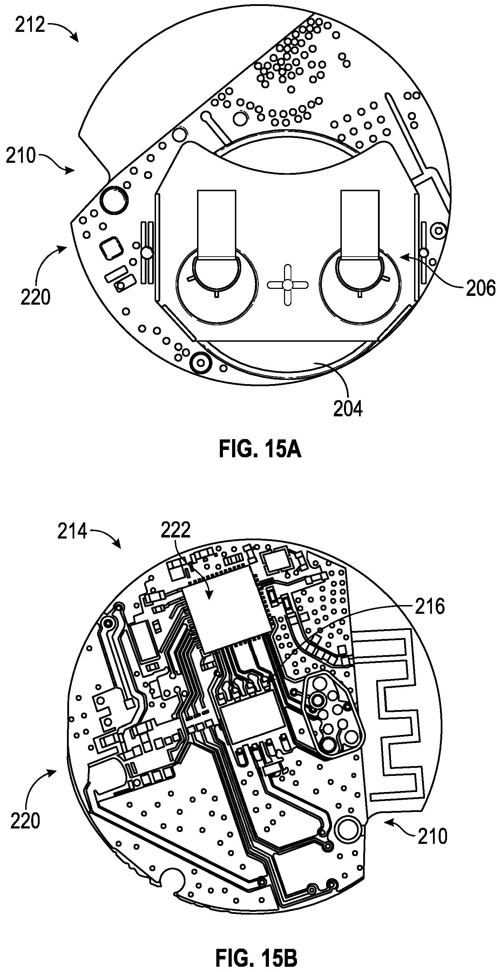

FIG. 15A illustrates the top side 212 of the PCB 152. As described above, the battery housing 206 may be attached to the top side 212. FIG. 15B illustrates a bottom side 214 of the PCB 152 in accordance with aspects of the present disclosure. The PCB 152 includes components coupled to the bottom side 214. The components may comprise a circuit 220 including resistors, LEDs, transistors, capacitors, inductors, transducers, diodes, switches, the sensor 216, a transmitter 222, or any other desired component. The components may be attached to the PCB 152 using a surface mount method, a through-hole method, or any other desired method. The PCB 152 may include additional and/or fewer components and is not limited to those illustrated in FIGS. 15A and B.

The circuit 220 may be configured to generate an electrical signal based on the rotation of the magnet 156. The circuit 220 may be configured to transmit the electrical signal in real time. The circuit 220 may transmit the electrical signal. For example, a transmitter 222 may be coupled to the PCB 152 and configured to transmit an electrical signal based on the rotation of the magnet 156 to an external device. The transmitter 222 may include wired or wireless transmission, such as Bluetooth^{™}, WiFi, NFC or any other means or method of desired transmission. The external device may be a mobile phone, a computer, a tablet, or any other desired device. The external device may have a user interface. The user interface may be configured to receive the electrical signal and display data obtained from the electrical signal. The data may include the angle of the joint 107, or any other desired information.

The user interface may include an app that receives the data, manipulates the data, records the data, and displays aspects of the data. For example, the app may display the angle of the joint 107 of a user 102, a history of the angle of the joint 107, duration of the angle, or any other desired information, such as a measurement of the angle in real time.

The sensor 216 may be a Hall Effect sensor, or any other desired sensor (e.g., a magnetic position sensor AS5601 using internal MEMS Hall Effect sensors). The sensor 216 may be coupled to the PCB 152 or any other desired device. The sensor 216 may be coupled to the bottom side 214 of the PCB 152 at a location directly above the magnet 156 when the PCB 152 is disposed within the center hub 116. The PCB 152 and the sensor 216 may rotate with the first hub 146 and the first arm 128. The magnet 156 may rotate with the second hub 148 and the second arm 130. The design of the wearable device 100, including the configuration of the sensor 216 and the magnet 156, may improve the accuracy of the measurements of the angle of the joint 107.

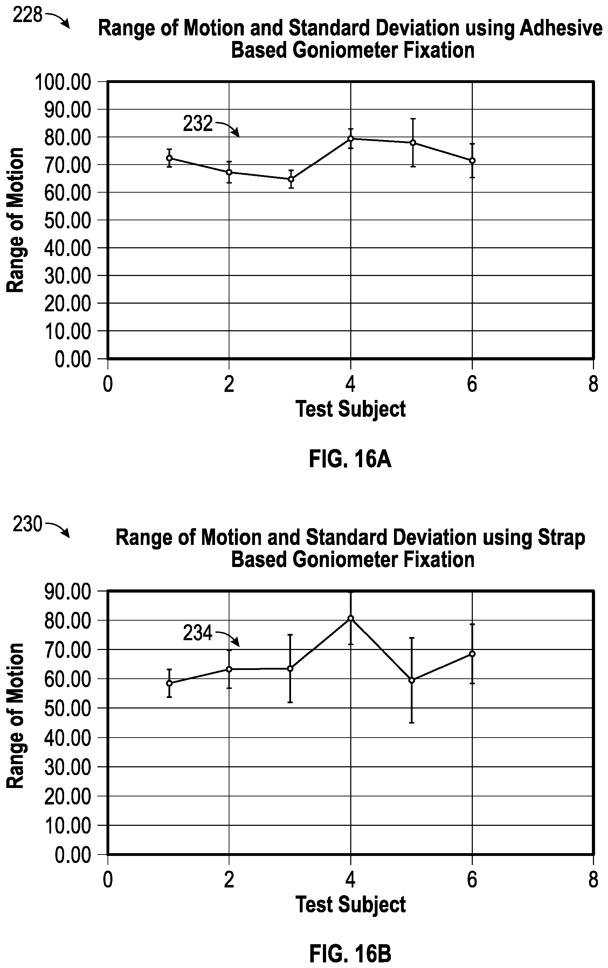

FIG. 16A is an exemplary graph 228 with line 232 depicting, while the wearable device 100 is attached to the user 102, the accuracy of measurements of angles of the joint 107 by the wearable device 100. FIG. 16B is an exemplary graph 230 with line 234 depicting the accuracy of measurements of angles of the joint 107 by a different measurement device attached to a user using Velcro^{™} straps. The standard deviation of the measurements made by the different measurement device shown by line 234 is greater than the standard deviation of the measurements made by the wearable device 100 shown by line 232. Users, such as clinicians or patients, are able to more accurately initially place and realign the first and second attachments 118 and 120 of the wearable device 100 as compared to using the Velcro^{™} straps. The configuration of the first and second arms 128, 130 of the wearable device 100 to fit different users may increase accuracy of the measurements. The configuration of the components, including the PCB 152, the sensor 216, and the magnet 156 within the center hub 116 of the wearable device 100 may further increase accuracy of the measurements. The wearable device 100 may be configured to measure the angle of the joint 107 up to an accuracy of measurement up to a one hundredth degree. The different measurement device may have an accuracy up to five degrees. In other words, the measurements taken by the different measurement device may have an accuracy of about +/- five degrees, such as about +/1 1 degree, or even +/- about 0.01 degree from the actual angle of the joint 107.

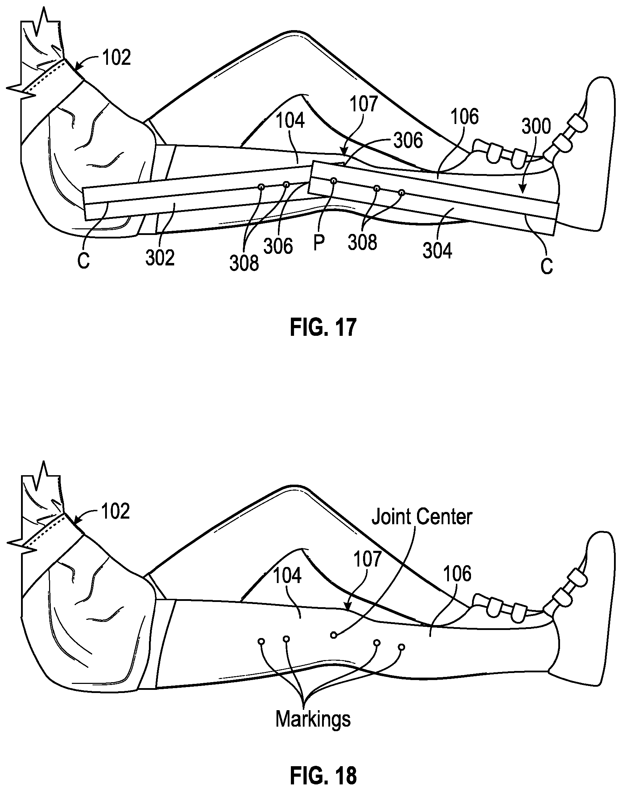

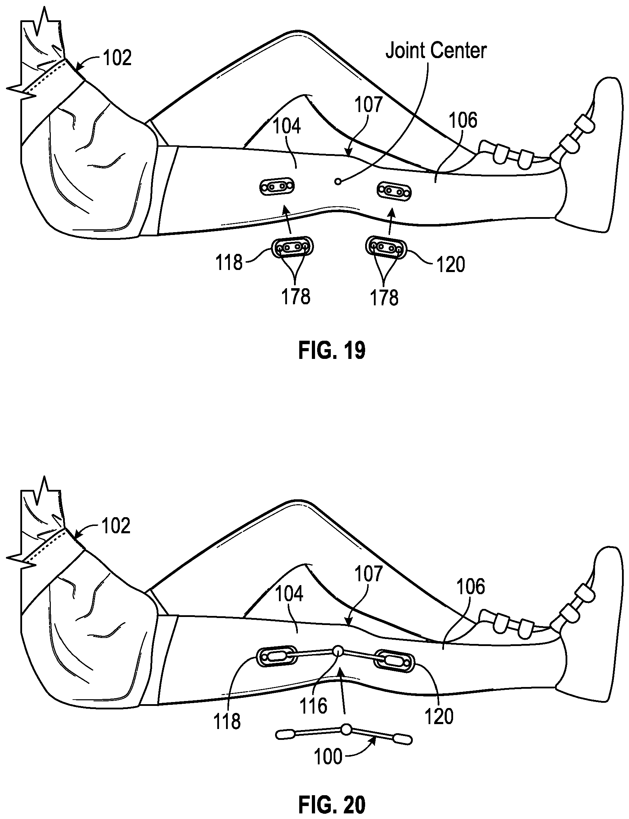

With reference to FIGS. 17 and 18, the alignment device 300 may be used to assist a clinician to align the wearable device 100 (*see* FIG. 20) on opposing limb 104, 106 portions of the joint 107 of the user 102 relative to a center of the joint 107. Properly aligning the wearable device 100 relative to the joint 107 facilitates greater accuracy and precision of the measurements made with the wearable device 100. Hence, the need for a device, such as alignment device 300, to assist in aligning the wearable device 100 relative to the joint 107 of a patient 102.

The alignment device 300 comprises a first segment 302 and a second segment 304 pivotably coupled to the first segment 302 at a pivot point P. More specifically, the segments 302, 304 each have a coupling end 306 where the segments 302, 304 pivotably couple to one another at the pivot point P. Moreover, the pivot point P is spaced adjacently and equidistant from each coupling end 306 of the segments 302, 304. Further yet, when the segments 302, 304 are pivotally coupled, center axes C of each segment 302, 304 intersect at the pivot point P.

The alignment device 300 further includes voids 308 defined by each segment 302, 304, and the voids 308 facilitate the marking of the skin of the user 102 during use of the alignment device 300. The voids 308 are spaced equidistant on each segment 302, 304 from the pivot point P, and the spacing between, and size, of each void 308 is commensurate with the spacing between, and size, of each alignment hole 178 of the attachments 118, 120 (*see* FIGS. 19 and 20). Further yet, when the attachments 118, 120 are coupled to the goniometer attachments 168, 170, the spacing between, and position of, each void 308 and the pivot point P is commensurate with the spacing between the center hub 116 and the alignment holes 178 of the attachments 118, 120.

Using the alignment device 300, the clinician may locate the center of the joint 107 of the user 102, and then position the pivot point P at the center of the joint 107. When the joint 107 of the user 102 is a knee, the clinician may locate the lateral epicondyle of the knee, and position the pivot point P adjacent to the lateral epicondyle. With the pivot point P positioned adjacent to the lateral epicondyle, the clinician may centrally position each segment 302, 304 adjacent to the opposing limb portions 104, 106 of the joint 107. The clinical may rely on the center axes C of each segment 302, 304 to assist in centrally aligning with the segments 302, 304 to the opposing limb portions 104, 106. With reference to FIGS. 17 and 18, with the segments 302, 304 centrally aligned with the opposing limb portions 104, 106, the clinician may use the voids 308 to mark the skin of the user 102. Thereafter, and with reference to FIGS. 19 and 20, the clinician may coaxially align the alignment holes 178 of the attachments 118, 120 with the markings on the skin of the user 102 to facilitate the alignment, and coupling to the user 102, of the wearable device 100 relative to the center of the joint 107. Further, the clinician may use pegs to assist in aligning with the attachments 118, 120 coaxially with the marks.

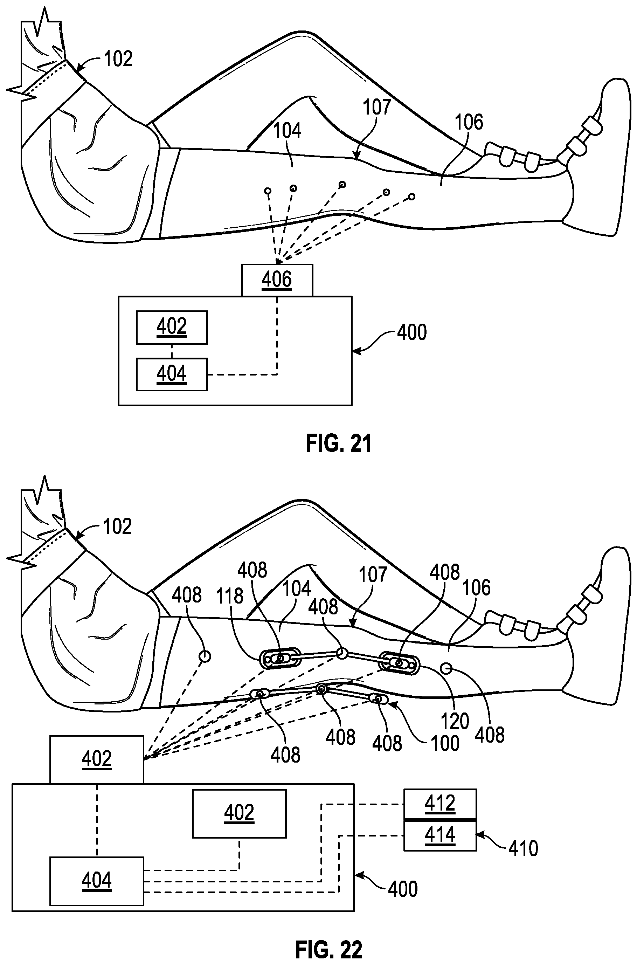

Alternatively, and with reference to FIGS. 21 and 22, an alignment device 400 may be used to assist a clinician to facilitate the alignment of the wearable device 100 relative to a center of the joint 107 of the user. More specifically, alignment device 400 may rely on data from an imaging device 402 of the joint 107 to assist the clinician in positioning the wearable device 100 on the user 102. The imaging device 402 may be an x-ray, ultrasound, any other imaging device capable of providing image data in 2D, 3D or 4D to the alignment device 400, or any other imaging device. A processor 404 of the alignment device 400 receives and processes the image data from the imaging device 402, and the processor 400 facilitates communication from the alignment device 400 to the clinician for the proper positioning of the attachments 118, 120, or the wearable device 100.

In one embodiment, and with reference to FIG. 21, the alignment device 400 may include one or more lasers 406 that produce a light beam. In this embodiment, the processor 404 may communicate with the lasers 406 to cause the lasers 406 to be positioned such that light beams causes spots of light on the skin of the user 102 on the center of the joint 107 and on opposing limb portion 104, 106. The spacing between, and size of, the spots of light are commensurate with the spacing between, and size of, each alignment hole 178 of the attachments 118, 120. The clinician may rely on the spots to align the wearable device 100 to center of the joint 107. Alternatively, the clinician may mark the locations of the spots of light on the skin, similar to the markings used with alignment device 300, and rely on the markings to align the wearable device 100 to the center of the joint 107. Further yet, the wearable device 100 may have indicia which the clinician may align with the light beams to facilitate alignment of the wearable device 100. In yet another alternative, the light beam may create an outline of the wearable device 100 on the skin of the user 102, where the outline may be commensurate to a perimeter of the wearable device, and may be used to properly align the wearable device 100 to the center of the joint 107.

In another embodiment of the alignment device 400, and with reference to FIG. 22, to assist the clinician in aligning the wearable device 100 relative to a center of the joint 107 of the user 102, the imaging device 402 may communicate with diodes 408 positioned on the user 102 and/or the wearable device 100. The diodes 408 may provide position data to the processor 404 such that the processor 404 may communicate with, and position, the lasers 406 to assist the clinician in the positioning of the attachments 118, 120. Alternatively, with the position data of the diodes 408, the processor 404 may communicate, to the clinician, with a clinician communication device 410, to assist in the alignment of the wearable device 100. Further yet, the wearable device 100 may also have diodes 408, which may communicate with the imaging device 402. Relying on position data of the diodes 408 from the imaging device 402, the processor 404 may cause the speaker 412 or the display 414 to provide respective audio or image outputs to communicate instructions, to the clinician, to facilitate the alignment of the wearable device 100. For example, the speaker 412 may provide audio guidance to the clinician, such as "move wearable device 5 mm to the left," to assist in the positioning of the wearable device 100. In another example, the display 414 may provide visual guidance to the user 107 on the location of the wearable device 100, and instructions for adjusting its position to achieve proper alignment. The display 414 may be a monitor, or iPad, or any other device for providing an image to the user, such as 3D googles. Of course, any of the above techniques could be used in combination with one another to assist the clinician to properly align the wearable device 100.

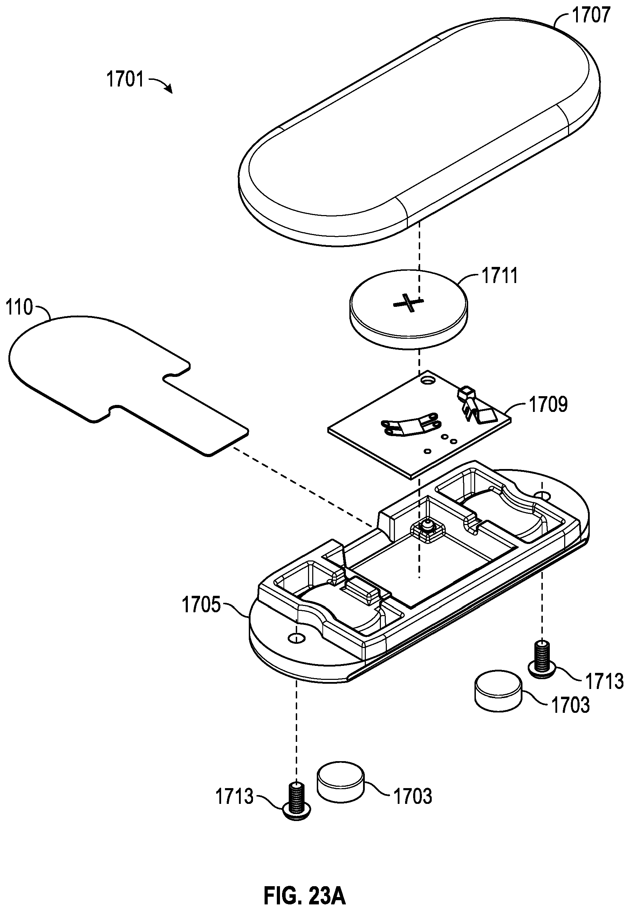

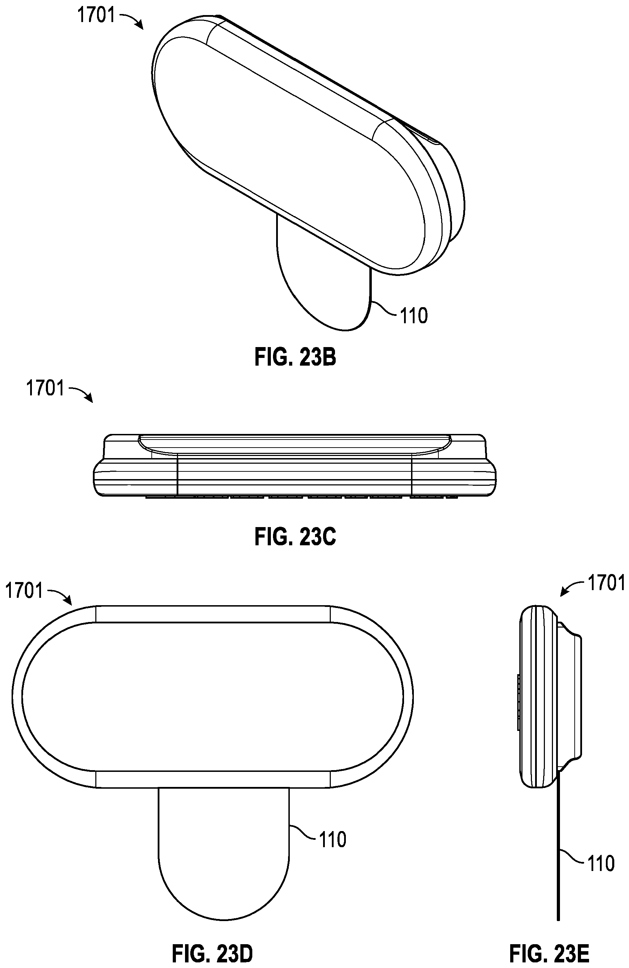

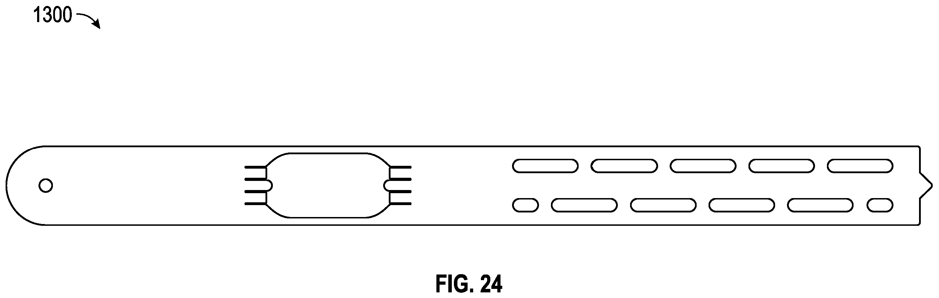

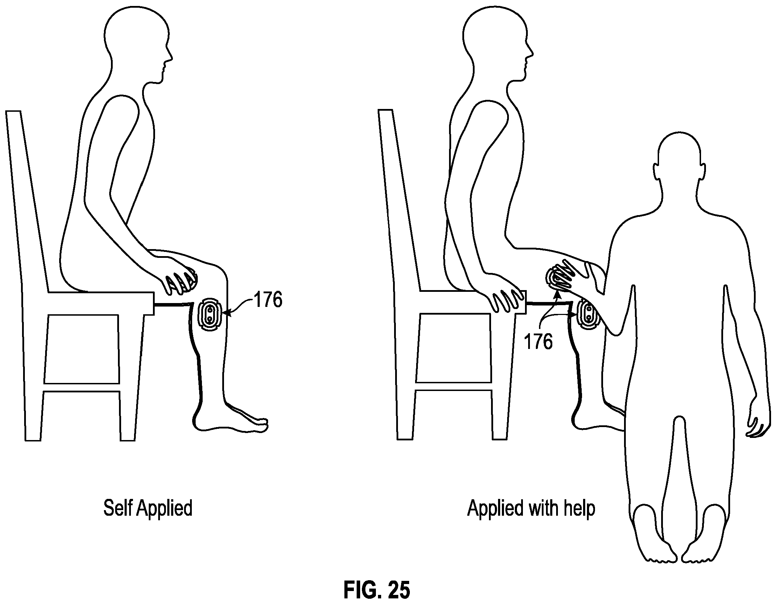

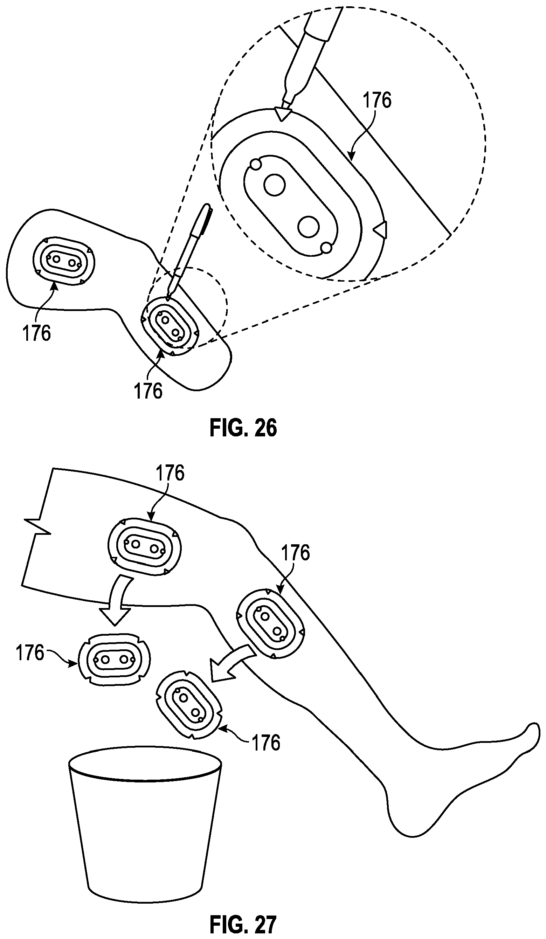

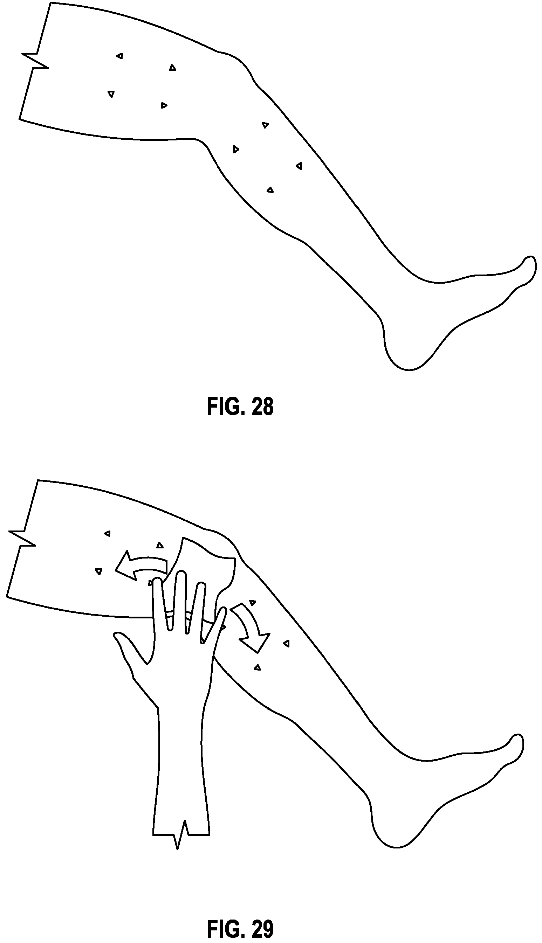

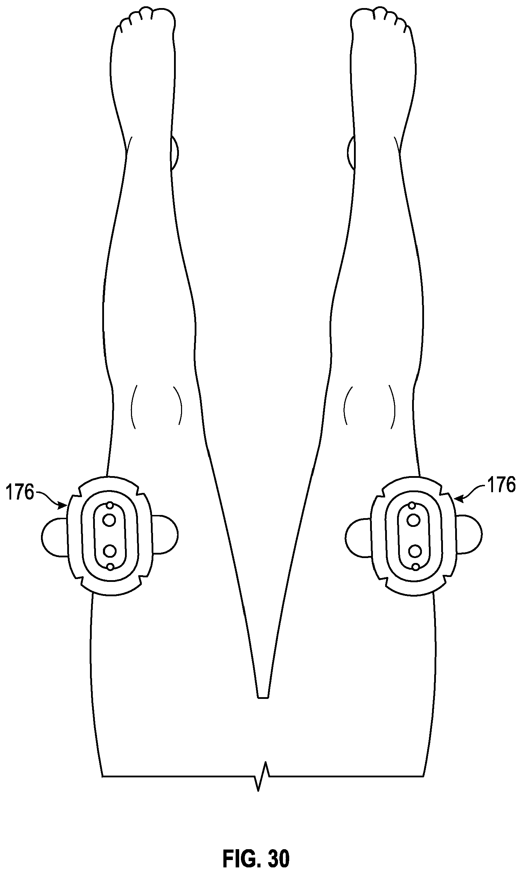

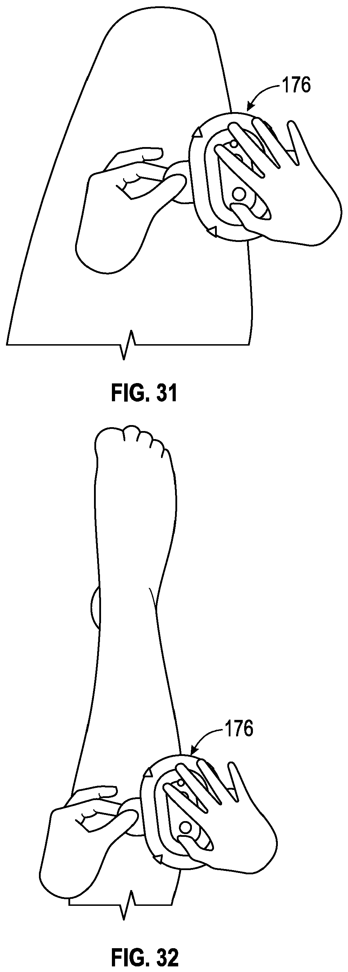

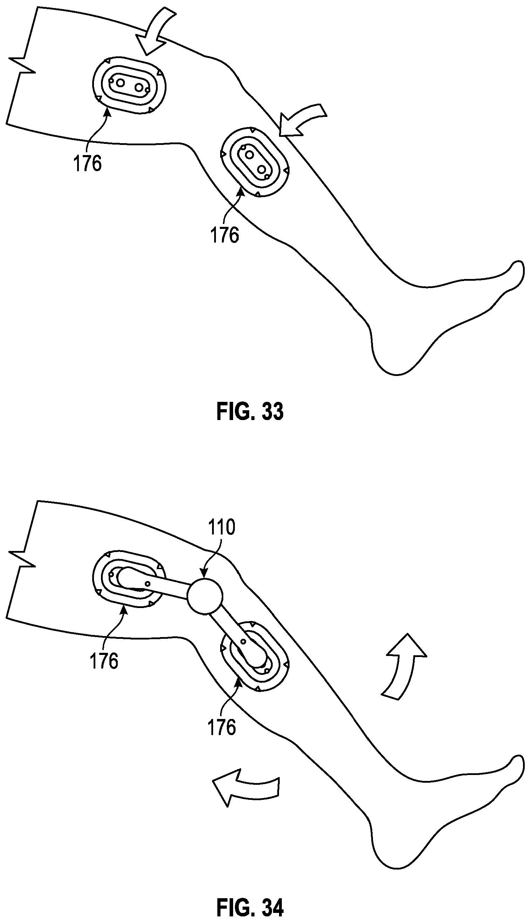

FIGS. 23A-23E depict an embodiment of an ambulation monitor or pedometer 1701. Versions of the pedometer 1701 can count the number of steps that a user takes, such as a daily count of steps post-operatively. Such a device can be carried by the user or attached to the user or to a peripheral of the user, such as the goniometer 110. The pedometer 1701 is operational to track steps of the user even if the user requires a walker or other assistance device.

In some versions, the pedometer 1701 can include the ability to attach to the magnets of the goniometer 110 to ensure accurate tracking of all steps of the user. The pedometer 1701 can include metallic elements that are magnetically attracted to the magnets of the goniometer 110. Alternatively, additional magnets 1703 mounted to the pedometer 1701. Embodiments of the pedometer 1701 can further include a body 1705, a removable cap 1707, a circuit board 1709 including one or more sensors (e.g., a motion sensor such as an accelerometer, mechanical sensor or other electromechanical sensor), a battery 1711 and fasteners 1713.

## Claims

1. A system (100) for measuring an angle of a joint (107) of a user (102), comprising:
a center hub (116), the center hub (116) having a first hub (146) and a second hub (148);
a first arm (128) configured for attachment to a first limb portion (104) of the user (102) at a first outer end (168) and to the first hub (146) at a first inner end (132);
a second arm (130) configured for attachment to a second limb portion (106) of the user (102) at a second outer end (170) and to the second hub (148) at a second inner end (134), wherein the first hub (146) is pivotally coupled to the second hub (148);
a magnet (156) coupled to the second hub (148); and
a sensor (216) disposed in the center hub (116) and configured to detect a rotation of the magnet (156),
**characterized in that** the first hub (146) and the second hub (148) are configured to rotate 360 degrees relative to each other, and
**in that** the system (100) further comprises:
a printed circuit board, PCB, (152) removably disposed in the center hub (116), the PCB (152) having the sensor (216) and an inward notch (210); and
an outward notch (208) coupled to the first hub (146), wherein the outward notch (208) is configured to couple with the inward notch (210) to align the sensor (216) and the magnet (156).

2. The system (100) of claim 1, further comprising a cover (150) detachably coupled to the first hub (146), wherein the cover (150) is configured to inhibit movement of the PCB (152) within the center hub (116).

3. The system (100) of claim 1, wherein the sensor (216) is a Hall Effect sensor coupled to the PCB (152).

4. The system (100) of claim 1, wherein the sensor (216) is configured to measure the rotation of the magnet (156) up to a precision of about +/- 0.01 degree, and/or wherein the system (100) further comprises a retaining ring (154) configured to couple the magnet (156) to the second hub (148), and/or wherein the magnet (156) is configured with north and south polarity within the center hub (116).

5. The system (100) of claim 1, further comprising a circuit (220) configured to generate an electrical signal based on the rotation of the magnet (156) and to transmit the electrical signal in real time.

6. The system (100) of claim 5, further comprising a user interface configured to receive the electrical signal and display data obtained from the electrical signal.

7. The system (100) of claim 6, wherein the data includes the angle of the joint (107) of the user (102).

8. The system (100) of claim 1, wherein the first hub (146) and the second hub (148) are configured to rotate 360 degrees about an axis (A).

9. The system (100) of claim 1, further comprising a battery housing (206) coupled to the PCB (152); and
a battery (204) detachably coupled to the battery housing (206).

10. The system (100) of claim 1, further comprising a transmitter (222) coupled to the PCB (152) and configured to transmit an electrical signal based on the rotation of the magnet (156).

11. The system (100) of claim 10, further comprising a user interface configured to receive the electrical signal and display data obtained from the electrical signal, wherein the data has the angle of the joint (107) of the user (102).

12. The system (100) of claim 1, wherein the first and second arms (128, 130) are adhesively attached to respective ones of the first and second limb portions (104, 106) of the user (102).

## Patentansprüche

1. System (100) zum Messen eines Winkels eines Gelenks (107) eines Benutzers (102), das Folgendes umfasst:
eine zentrale Nabe (116), wobei die zentrale Nabe (116) eine erste Nabe (146) und eine zweite Nabe (148) aufweist;
einen ersten Arm (128), der für das Anbringen an einem ersten äußeren Ende (168) an einem ersten Gliedmaßenabschnitt (104) des Benutzers (102) und an einem ersten inneren Ende (132) an der ersten Nabe (146) ausgebildet ist;
einen zweiten Arm (130), der so ausgebildet ist, dass er an einem zweiten äußeren Ende (170) an einem zweiten Gliedmaßenabschnitt (106) des Benutzers (102) und an einem zweiten inneren Ende (134) an der zweiten Nabe (148) angebracht werden kann, wobei die erste Nabe (146) schwenkbar mit der zweiten Nabe (148) gekoppelt ist;
einen Magneten (156), der mit der zweiten Nabe (148) gekoppelt ist; und
einen Sensor (216), der in der zentralen Nabe (116) angeordnet und für die Erkennung einer Drehung des Magneten (156) ausgebildet ist,
**dadurch gekennzeichnet, dass** die erste Nabe (146) und die zweite Nabe (148) für etwas ausgebildet sind, so dass sie sich relativ zueinander um 360 Grad drehen können, und
dadurch, dass das System (100) ferner Folgendes umfasst:
eine Leiterplatte (PCB) (152), die herausnehmbar in der zentralen Nabe (116) angeordnet ist, wobei die Leiterplatte (152) den Sensor (216) und eine nach innen gerichtete Kerbe (210) aufweist; und
eine nach außen gerichtete Kerbe (208), die mit der ersten Nabe (146) gekoppelt ist, wobei die nach außen gerichtete Kerbe (208) so ausgebildet ist, dass sie mit der nach innen gerichteten Kerbe (210) gekoppelt wird, um den Sensor (216) und den Magneten (156) auszurichten.

2. System (100) nach Anspruch 1, das ferner eine Abdeckung (150) umfasst, die lösbar mit der ersten Nabe (146) gekoppelt ist, wobei die Abdeckung (150) so ausgebildet ist, dass sie eine Bewegung der Leiterplatte (152) innerhalb der zentralen Nabe (116) verhindert.

3. System (100) nach Anspruch 1, wobei der Sensor (216) ein Hall-Effekt-Sensor ist, der an die Leiterplatte (152) gekoppelt ist.

4. System (100) nach Anspruch 1, wobei der Sensor (216) dafür ausgebildet ist, die Drehung des Magneten (156) mit einer Genauigkeit von etwa +/- 0,01 Grad zu messen, und/oder wobei das System (100) ferner einen Haltering (154) umfasst, der dafür ausgebildet ist, den Magneten (156) mit der zweiten Nabe (148) zu koppeln, und/oder wobei der Magnet (156) innerhalb der zentralen Nabe (116) mit Nord- und Südpol ausgebildet ist.

5. System (100) nach Anspruch 1, das ferner eine Schaltung (220) umfasst, die für die Erzeugung eines elektrischen Signals auf der Grundlage der Drehung des Magneten (156) ausgebildet ist und dieses elektrische Signal in Echtzeit überträgt.

6. System (100) nach Anspruch 5, das ferner eine Benutzerschnittstelle umfasst, die dafür ausgebildet ist, das elektrische Signal zu empfangen und aus dem elektrischen Signal gewonnene Daten anzuzeigen.

7. System (100) nach Anspruch 6, wobei die Daten den Winkel des Gelenks (107) des Benutzers (102) einschließen.

8. System (100) nach Anspruch 1, wobei die erste Nabe (146) und die zweite Nabe (148) dafür ausgebildet sind, dass sie sich um 360 Grad um eine Achse (A) drehen können.

9. System (100) nach Anspruch 1, das ferner ein mit der Leiterplatte (152) gekoppeltes Batteriegehäuse (206) umfasst; und
eine Batterie (204), die abnehmbar mit dem Batteriegehäuse (206) gekoppelt ist.

10. System (100) nach Anspruch 1, das ferner einen Sender (222) umfasst, der mit der Leiterplatte (152) gekoppelt ist und für die Übertragung eines elektrischen Signals auf der Grundlage der Drehung des Magneten (156) ausgebildet ist.

11. System (100) nach Anspruch 10, das ferner eine Benutzerschnittstelle umfasst, die für das Empfangen des elektrischen Signals ausgebildet ist und aus dem elektrischen Signal gewonnene Daten anzeigt, wobei die Daten den Winkel des Gelenks (107) des Benutzers (102) enthalten.

12. System (100) nach Anspruch 1, wobei der erste und der zweite Arm (128, 130) an den jeweiligen ersten bzw. zweiten Gliedmaßenabschnitten (104, 106) des Benutzers (102) angebracht sind.

## Revendications

1. Système (100) de mesure d'un angle d'une articulation (107) d'un utilisateur (102), comportant :
un moyeu central (116), le moyeu central (116) ayant un premier moyeu (146) et un deuxième moyeu (148) ;
un premier bras (128) configuré pour être fixé à une première partie de membre (104) de l'utilisateur (102) au niveau d'une première extrémité externe (168) et au premier moyeu (146) au niveau d'une première extrémité interne (132) ;
un deuxième bras (130) configuré pour être fixé à une deuxième partie de membre (106) de l'utilisateur (102) au niveau d'une deuxième extrémité externe (170) et au deuxième moyeu (148) au niveau d'une deuxième extrémité interne (134), le premier moyeu (146) étant couplé de manière pivotante au deuxième moyeu (148) ;
un aimant (156) couplé au deuxième moyeu (148) ; et
un capteur (216) disposé dans le moyeu central (116) et configuré pour détecter une rotation de l'aimant (156),
**caractérisé en ce que** le premier moyeu (146) et le deuxième moyeu (148) sont configurés pour tourner sur 360 degrés l'un par rapport à l'autre, et
**en ce que** le système (100) comprenant en outre :
une carte de circuit imprimé, PCB (152), disposée de manière amovible dans le moyeu central (116), la PCB (152) ayant le capteur (216) et une encoche vers l'intérieur (210) ; et
une encoche extérieure (208) couplée au premier moyeu (146), l'encoche extérieure (208) étant configurée pour être couplée à l'encoche intérieure (210) afin d'aligner le capteur (216) et l'aimant (156).

2. Système (100) selon la revendication 1, comprenant en outre un couvercle (150) couplé de manière amovible au premier moyeu (146), le couvercle (150) étant configuré pour empêcher la PCB (152) de bouger à l'intérieur du moyeu central (116).

3. Système (100) selon la revendication 1, dans lequel le capteur (216) est un capteur à effet Hall couplé à la PCB (152).

4. Système (100) selon la revendication 1, dans lequel le capteur (216) est configuré pour mesurer la rotation de l'aimant (156) jusqu'à une précision d'environ +/- 0,01 degrés, et/ou dans lequel le système (100) comprend en outre un anneau de retenue (154) configuré pour coupler l'aimant (156) au deuxième moyeu (148), et/ou dans lequel l'aimant (156) est configuré avec une polarité nord et sud à l'intérieur du moyeu central (116).

5. Système (100) selon la revendication 1, comprenant en outre un circuit (220) configuré pour générer un signal électrique basé sur la rotation de l'aimant (156) et pour transmettre le signal électrique en temps réel.

6. Système (100) selon la revendication 5, comprenant en outre une interface utilisateur configurée pour recevoir le signal électrique et afficher des données obtenues à partir du signal électrique.

7. Système (100) selon la revendication 6, dans lequel les données comprennent l'angle de l'articulation (107) de l'utilisateur (102).

8. Système (100) selon la revendication 1, dans lequel le premier moyeu (146) et le deuxième moyeu (148) sont configurés pour tourner de 360 degrés autour d'un axe (A).

9. Système (100) selon la revendication 1, comprenant en outre un boîtier de batterie (206) couplé à la PCB (152) ; et
une batterie (204) couplée de manière amovible au boîtier de batterie (206).

10. Système (100) selon la revendication 1, comprenant en outre un émetteur (222) couplé à la PCB (152) et configuré pour transmettre un signal électrique basé sur la rotation de l'aimant (156).

11. Système (100) selon la revendication 10, comprenant en outre une interface utilisateur configurée pour recevoir le signal électrique et afficher des données obtenues à partir du signal électrique, les données incluant l'angle de l'articulation (107) de l'utilisateur (102).

12. Système (100) selon la revendication 1, dans lequel les premier et deuxième bras (128, 130) sont fixés de manière adhésive à des parties respectives des première et deuxième parties de membre (104, 106) de l'utilisateur (102).
